# EUROPEAN PATENT SPECIFICATION

(11) **EP 1 526 860 B1**
(45) Date of publication and mention of the grant of the patent: **12.09.2007**
(21) Application number: 03741062.8
(22) Date of filing: 18.07.2003
(51) Int. Cl.: A61K 36/23, A61K 31/7048, A61K 47/12

(54) **BIOAVAILABILITY/BIOEFFICACY ENHANCING ACTIVITY OF CUMINUM CYMINUM AND EXTRACTS AND FRACTIONS THEREOF**
STEIGERUNG DER BIOVERFÜGBARKEIT/BIOEFFEKTIVITÄT DURCH CUMINUM CYMINUM UND DESSEN EXTRAKTE UND FRAKTIONEN
NOUVEAUX AGENTS A BASE DE PLANTES UTILISES COMME RENFORCATEURS DE LA BIODISPONIBILITE/BIOEFFICACITE DE MEDICAMENTS ET DE NUTRACEUTIQUES

(30) Priority: 18.07.2002 US 396654 P
(43) Date of publication of application: 04.05.2005
(73) Proprietor: COUNCIL OF SCIENTIFIC AND INDUSTRIAL RESEARCH, New Delhi 110 001 (IN)
(72) Inventor: QAZI, Ghulam, Nabi, 180 001 Jammu (IN); BEDI, Kasturi, Lal, 180 001 Jammu (IN); JOHRI, Rakesh, Kamal, 180 001 Jammu (IN); SHARMA, Subhash, Chander, 180 001 Jammu (IN); TIKOO, Manoj, Kumar, 180 001 Jammu (IN); TIKOO, Ashok, Kumar, 180 001 Jammu (IN); ABDULLAH, Sheikh, Tasaduq, 180 001 Jammu (IN); SINGH, Kuldeep, 180 001 Jammu (IN); PANDITA, Rashmi, 180 001 Jammu (IN); SURI, Om, Parkash, 180 001 Jammu (IN); GUPTA, Bisham, Datt, 180 001 Jammu (IN); SURI, Krishan, Avtar, 180 001 Jammu (IN); SATTI, Naresh, Kumar, 180 001 Jammu (IN); KHAJURIA, Ravi, Kant, 180 001 Jammu (IN); KAPAHI, Bal, Krishan, 180 001 Jammu (IN); KALSOTRA, Ashok, Kumar, 180 001 Jammu (IN); VERMA, Neelam, 180 001 Jammu (IN); ANAND, Rajneesh, 180 001 Jammu (IN)
(74) Representative: Fairbairn, Angus Chisholm
(86) International application number: PCT/IN2003/000244
(87) International publication number: WO 2004/009061

(56) References cited:
- EP-A- 1 031 348
- WO-A-03/075685
- US-A1- 2001 055 625
- EL-SHOBAKI F A ET AL: "THE EFFECT OF SOME BEVERAGE EXTRACTS ON INTESTINAL IRON ABSORPTION" ZEITSCHRIFT FUER ERNAEHRUNGSWISSENSCHAFT, STEINKOPF VERLAG, DARMSTADT, DE, vol. 29, no. 4, 1990, pages 264-269, XP009015757 ISSN: 0044-264X
- PLATEL K ET AL: "INFLUENCE OF DIETARY SPICES OR THEIR ACTIVE PRINCIPLES ON DIGESTIVE ENZYMES OF SMALL INTESTINAL MUCOSA IN RATS" INTERNATIONAL JOURNAL OF FOOD SCIENCES AND NUTRITION, CARFAX PUBLISHING LTD, GB, vol. 47, no. 1, 1996, pages 55-59, XP008016081 ISSN: 0963-7486

## Description

### Technical Field

The present invention is directed to a composition containing an active molecule from the plant *Cuminum cyminum* and methods of using such molecule to enhance bioavailability of drugs, natural products and essential nutraceuticals. The present invention is intended to enhance the bioavailability/ bioefficacy of drugs which are poorly bioavailable or given for a long period of time and are expensive and toxic.

There is a great interest and medical need for the improvement of bioavailability of a large number of drugs which are (a) poorly bioavailable, (b) given for long periods, and are (c) toxic and expensive. Maximizing oral bioavailability is therapeutically important because the extent of bioavailability directly influences plasma concentrations as well as therapeutic and toxic effects resulting after oral drug administration. Poorly bioavailable drugs remain sub-therapeutic because a major portion of a dose never reaches the plasma or exerts its pharmacological effect unless and until very large doses are given which may lead to serious side effects. Any significant improvement in bioavailability will result in lowering the dose or the dose frequency of that particular drug. Besides, inter-subject variability is inversely correlated with the extent of bioavailability. Therefore, low oral bioavailability leads to high variability and poor control of plasma concentration and pharmacodynamic effects. Inter-subject variability is particularly of concern for a drug with a narrow safety margin.

Incomplete oral bioavailability has various causes. These include poor dissolution or low aqueous solubility, poor intestinal membrane permeation, degradation of the drug in gastric or intestinal fluids and pre-systemic intestinal or hepatic metabolism. The normal practice to offset some of these problems has been to increase the dosage as stated earlier which has the concerns of patients' non-compliance and toxicity.

Many therapeutic treatments are also accompanied by loss of essential nutraceuticals in the course of therapy. The present invention improves nutritional status by increasing bioavailability/ bioefficacy of various nutraceuticals also which include metals and vitamins.

### Background Art

Several approaches have been adopted in the past to maximize oral bioavailability, such as (a) micronization, (b) polymorphic or crystal size and form selection, (c) solubilization of lesser soluble drugs by way of chemical modifications, complexation and use of co-solvents/ surfactants, (d) targeted delivery of drug at the site of action, (e) controlled drug delivery by film coating or use of polymeric matrices for sustained release of drugs, (f) prodrug approach, and (g) microencapsulation using liposomes.

However, based on clues from Ayurvedic literature, a new approach of increasing the bioavailability of drugs including poorly bioavailable drugs had been conceptualized at RRL Jammu. One of the groups of herbals which has been documented very frequently as essential part of about 70 % of Ayurvedic prescriptions, was noted to be 'Trikatu', that comprises the three acrids viz. long pepper, black pepper and dry ginger in equal proportions. A single major alkaloidal constituent from peppers (piperine) was found to be responsible for bioavailability enhancing effect. Influence of piperine was extensively studied on anti-TB drugs. It was determined that in combination with piperine the dose of rifampicin can be reduced by about 50% while retaining the therapeutic efficacy of this anti-TB drug at par with the standard dose (450 mg). Based on these findings several other reputed plants were evaluated for bioavailability/ bioefficacy enhancing activity. Polar and non-polar extracts of parts of a few plants viz., *Zingiber officinalis, Carum carvi and Cuminum cyminum* increased significantly (25 - 300 %), the bioavailability of a number of classes of drugs, for example, but not limited to, antibiotics, antifungals, anti-virals, anticancer, cardiovascular, CNS, anti-inflammatory/anti-arthritic, anti-TB/antileprosy, antihistaminic/, corticosteroids, immunosppressants. Such extracts either in presence or absence of piperine have been found to be highly selective in their bioavailability/bioefficacy enhancing action.

### Description of the preferred embodiment

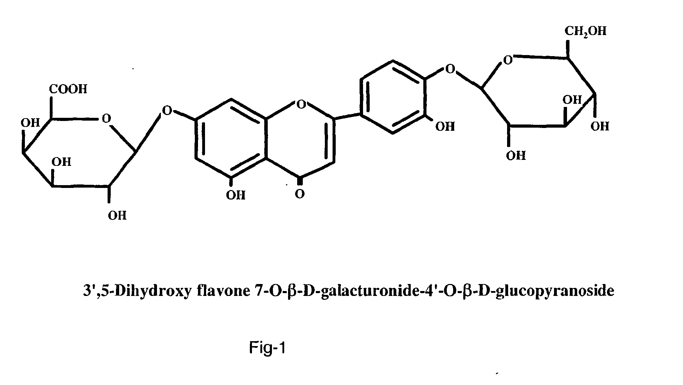

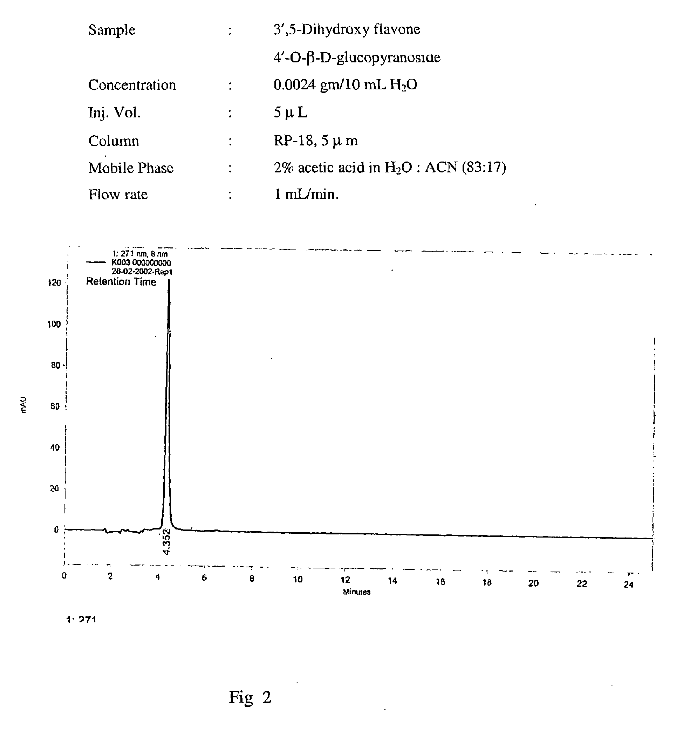

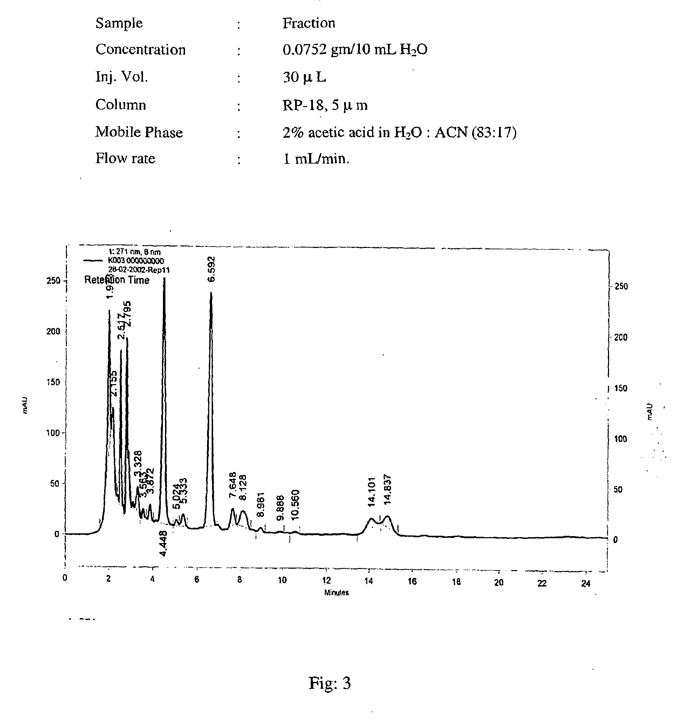

The present invention is directed to a composition containing an active molecule from the plant *Cuminum cyminum* and methods of using such molecule to enhance bioavailability of drugs, natural products and essential nutraceuticals. The active molecule enhances bioavailability/ bioefficacy of certain drugs, natural products and essential nutraceuticals. The chemical name and structure of the active molecule is shown in Fig 1. HPLC fingerprint of the active molecule and the fraction is shown in Fig 2 and 3 respectively. The compound (Fig 1) though known has been for the first time reported to be useful as an effective bioavailability enhancer

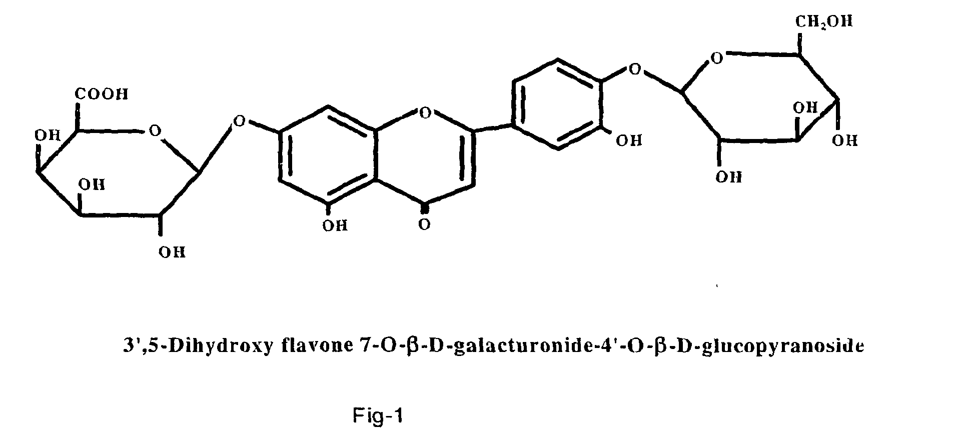

| | |
|---|---|
| Sample : | 3',5-Dihydroxy flavone-7-O-β-D-galacturonide-4'-O-β-D-glucopyranoside |
| Concentration : | 0.0024 gm/10 mL H₂O |
| Inj. Vol. : | 5 µL |
| Column : | RP-18,5 µm |
| Mobile Phase : | 2% acetic acid in H₂O : ACN (83:17) |

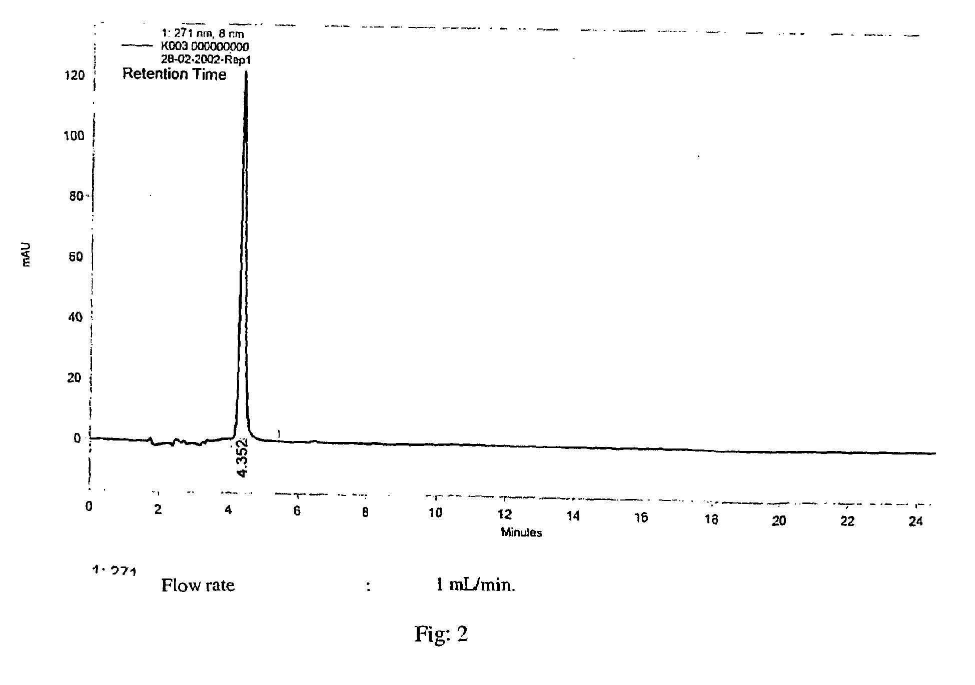

| | |
|---|---|
| Sample : | Fraction |
| Concentration : | 0.0752 gm/10 mL H₂O |
| Inj. Vol. : | 30 µL |
| Column : | RP-18, 5 µm |
| Mobile Phase : | 2% acetic acid in H₂O : ACN (83:17) |
| Flow rate : | 1 mL/min. |

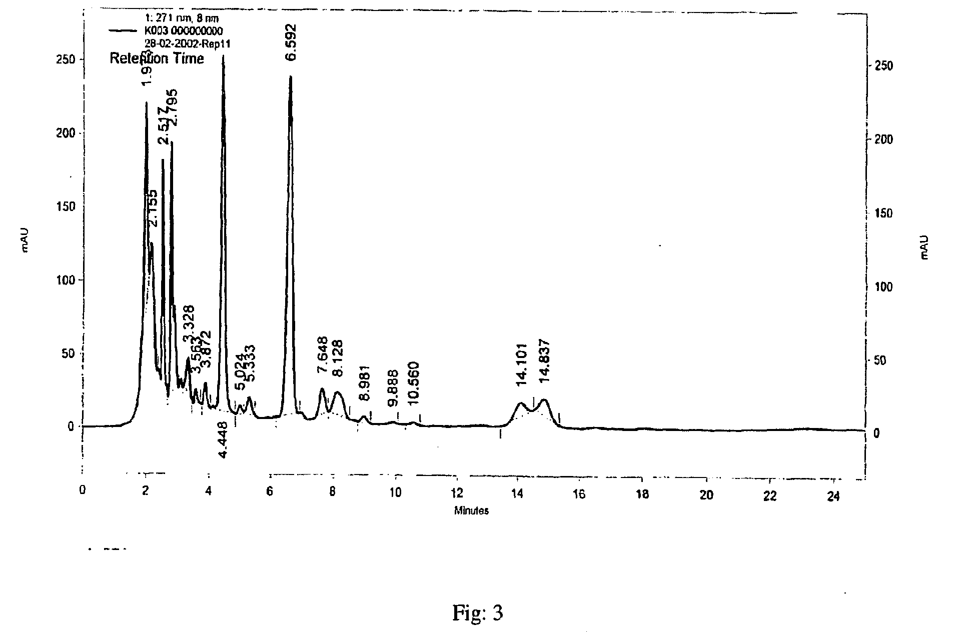

Greenish yellow powder (H₂O:EtOH), soluble in H₂O , m.p. 270°C decompose.

### UV λmax. nm

| | |
|---|---|
| MeOH | 256.5, 267.5 sh, 350 |
| NaOMe | 265.5, 393, 5 |
| AlCl₃ | 273, 327.5 sh, 429.5 |

Physical and chemical data of as 3',5-Dihydroxy flavone-7-O-β-D-galacturonide-4'-O-β-D-glucopyranoside
Greenish yellow powder (H₂O:EtOH), soluble in H₂O, m.p. 270°C decompose.

### UV λmax. nm

| | |
|---|---|
| MeOH | 256.5, 267.5 sh, 350 |
| NaOMe | 265.5, 93,5 |
| AlCl₃ | 273, 327.5 sh, 429.5 |
| AlCl₃ / HCl | 266, 358 |
| NaOAc | 261.5,406.5 |
| NaOAc/H₃BO₃ | 260, 374.5 |

### ¹HNMR (DM SO-d6):

δ 3.08-3.75 (m, 17H, sugar protons), 4.50 (d, 1H, J= 7.21 Hz, H-1"'), 5.21 (d, 1H, J= 6.82 Hz, H-1"), 6.42 (bs, 1H, H-6), 6.65 (bs, 1H, H-8), 6.81 (d, 1H, J=8.42, H-5'), 7.09 (s, 1H, H-3), 7.35 (q, 1H, J= 8.42 and 1.8 Hz, H-6'), 7.80 (bs, 1H, H-2').

### ¹³CNMR (H₂O-CD₃OD) :

δ 165.36 (C-2) , 104.01 (C-3), 183.19 (C-4), 160.87 (C-5), 99.41 (C-6), 163.09 (C-7), 96.31 (C-8), 157.65 (C-9), 106.50 (C-10), 122.36 (C-1') ,114.10 (C-2'), 145.37 (C-3 '), 148.74 (C-4'),116.81 (C-5'), 120.75 (C-6'), 101.21 (C-1"), 73.25 (C-2"), 77.23 (C-3"), 70.72 (C-4"), 76.89 (C-5"), 62.02 (C-6"), 103.39 (C-1"'), 75.02 (C-2"' and C-4"'), 77.71 (C-3 "') 82.07 (C-5"'),176.44 (C-6"').

On the basis of above data the compound has been identified as 3',5-Dihydroxy flavone-7-O-β-D-galacturonide-4'-O-β-D-glucopyranoside (Fig. 1).

The compound acts by any one or more than one of the following ways: (a) Promoting the absorption of drugs from GIT, (b) Inhibiting or reducing the rate of biotransformation of drugs in the liver or intestines, (c) Modifying the immune system in a way that the overall requirement of the drug is reduced substantially, (d) Increasing the penetration or the entry into the pathogens even where they become persistors within the macrophages such as for *Mycobacterium tuberculosis* and such others. This eventually ensures the enhanced killing of these organisms well secured within the places otherwise inaccessible to the active drug. (e) Inhibiting the capability of pathogens or abnormal tissue to reject the drug e.g., efflux mechanisms frequently encountered with anti-malarial, anti-cancer and anti-microbial drugs, (f) Modifying the signalling process between host and pathogen ensuring increased accessibility of the drugs to the pathogens, (g) Enhancing the binding of the drug with the receptors like proteins, DNA, RNA, etc., in the pathogen, thus potentiating and prolonging its effect leading to enhanced antibiotic activity against pathogens, (h) Besides above plausible modes of action, the bioenhancer agents may also be useful for promoting the transport of nutrients and the drugs across the blood brain barrier, which could be of immense help in the control of diseases like cerebral infections, epilepsy and other CNS problems.

Primarily, but not exclusively, the invention enhances the carrier mediated entry of drugs and also the passive diffusion and the active transport pathways in the tissue which are responsible for transporting physiological substances such as nutraceuticals to their target sites. As applicable to any mechanism of action the compound contributes in a synergistic and /or additive manner so that most drugs and nutraceuticals in presence of the compound described in the present art are more bioavailable as a result of one or more of these mechanisms. As a preferred embodiment, the active molecule increases the plasma levels and bioefficacy of certain categories of drugs and nutraceuticals by 80 - 220 % over the effect that results from normal intake of therapeutic and nutraceutical products.

The ratio (w/w) of an effective bioenhancer (active molecule) in combination with a drug/nutraceutical may vary in the range of 0.1 to 300 %.

The bioavailability of drugs and nutraceuticals is also relevant to animal health besides being important for humans. The invention therefore is also intended to be used in veterinary preparations.

### Brief description of the accompanying drawings:

**Fig.1** shows the active molecule 3',5-dihydroxy flavone 7-O-β-D-galacturonide-4'O-β-D-glucopyranoside.
**Fig.2** shows the HPLC fingerprint of the said active molecule 3',5-dihydroxy flavone 7-O-β-D-galacturonide-4'O-β-D-glucopyranoside.
**Fig.3** shows the HPLC fingerprint of the fraction isolated from plant *Cuminum cyminum.*

**EXAMPLES:** The following examples are intended to demonstrate some of the preferred embodiments and in no way should be construed so as to limit the scope of the invention. Any person skilled in the art can design more formulations, which may be considered as part of the present invention.

### Example 1:

*Cuminum cyminum* seeds (0.5 kg) were ground to a coarse powder and then extracted with deionised water at 98± 1°C for 2 hrs. Extraction process was repeated four times using total of 3.1 litres water (1 x 1 litre + 3 x 0.7 Litre, four extractions). All the four extracts were pooled. The pooled extract was centrifuged, followed by vacuum filtration through a celite bed. The clear filtrate was lyophilized to get greenish yellow amorphous powder (yield 88 gm, 17. 6 %). The dry extract was dissolved in deionised water (500 mL) and partitioned between n-BuOH (6 x 500 mL) and H₂O. The n-BuOH extract was concentrated on a rotavapour under reduced pressure at 65°C (residue 11.0 gm). n-BuOH free aqueous extract was freeze dried (residue 75.0 gm) and subjected to adsorption chromatography. Aqueous extract residue was dissolved in minimum quantity of H₂O and adsorbed on SiO₂ gel, 60-120 mesh (150 gm). Solvent was completely removed to get free flowing material. A glass column of 1.5 inch dia was packed with 100 gm SiO₂ gel, 60-120 mesh in EtOAc. The adsorbed material was charged in the column over the packed SiO₂ gel. The column was eluted with EtOAc and then with EtOH by gradually increasing the percentage of H₂O in EtOH. In all 420 fractions of 70 mL each were collected and pooled on the basis of TLC pattern using BuOH (B) : AcOH (A) : H₂O (W) (4:1:5) as developing solvent. Spots were visualized by spraying with freshly prepared Borinate-PEG solution [ 1% solution of 2-aminoethyl diphenylborinate in MeOH and 5% solution of polyethylene glycol 4000 in EtOH (mixed 1:1 v/v before spraying)]. Fraction no. 81-167 (eluted in EtOH and 10% H₂O in EtOH) showed same TLC pattern. These fractions were pooled, dried and then dissolved in minimum quantity of water. Crystallisation was carried out by the addition of EtOH in small portions, supernatant was drained off and residue was washed with aq. EtOH. Residue was repeatedly crystallized from H₂O : EtOH. A yellow powder (70 mg) soluble in H₂O was thus obtained. Compound Rf 0.28 solvent system B : A : W (4:1:5) was identified as 3',5 -dihydroxy flavone 7-O-β-D-galacturonide-4'-O-β-D-glucopyranoside.

### Example 2

*Cuminum cyminum* seeds (0.5 kg) were ground to a coarse powder. The powder was soaked in 50% aqueous ethanol (1.0 L) for 16 hrs. The marc was extracted three times more under same conditions using 0.7 L of extraction solvent each time. The pooled extract was clarified by vacuum filtration through a celite bed. The extract thus obtained was concentrated at 60 ± 2°C on a rotavapour. The EtOH free extract was lyophilized to get a greenish yellow powder (88 gm, 17.60%). 80 gm of the extract was extracted by heating on a steam bath respectively with

| | | |
|---|---|---|
| 1. | CHCl₃ | (2 X 200 mL) |
| 2. | 10% EtOH in CHCl₃ | (1x 200) |
| 3. | 20% EtOH in CHCl₃ | (1x 200 mL) |
| 4. | 30% EtOH in CHCl₃ | (1x 200 mL) |
| 5. | 40% EtOH in CHCl₃ | (1x 200 mL) |
| 6. | 50% EtOH in CHCl₃ | (1x 200 mL) |
| 7. | 60% EtOH in CHCl₃ | (1x 200 mL) |
| 8. | 70% EtOH in CHCl₃ | (1x 200 mL) |
| 9. | EtOH | (6 x 200 mL) |
| 10. | EtOH + 10% H₂O | (1 x 200 mL) |

The insoluble residue left after extraction with 10% water in EtOH was then extracted at room temperature with EtOH + 20% H₂O (3 x 500 mL). The left over fraction (25 gm) was subjected to adsorption chromatography. It was adsorbed on silica gel (60-120 mesh 70 gm). Solvent was completely removed to get free flowing material. A glass column of 1.5 inch dia was packed with 70 gm SiO₂ gel 60-120 mesh in EtOH. The adsorbed extract was charged in the column. The column was eluted with EtOH by gradually increasing the %age of H₂O. In all 94 fractions of 70 mL each were collected and pooled on the basis of TLC pattern using B:A:W (4:1:5) as developing solvent. Spots were visualized by spraying the TLC plate with Borinate PEG spray reagent. Fractions 56-80 homogeneous on TLC were pooled, dried and charged on a Sephadex LH-20 column. Column was eluted with water then with EtOH to produce two fractions of 200 and 500 mL respectively. The first fraction was purified repeatedly (three times) on Sephadex LH-20 column. TLC homogeneous fractions containing target compound were pooled and residue was repeatedly crystallized from H₂O : EtOH. A yellow powder (50 mg) soluble in water was obtained. Compound Rf 0.28 , Solvent system : B : A : W (4 : 1 : 5) was identified 3',5-dihydroxyflavone 7-O-β-D-galacturonide-4'-O-β-D-glucopyranoside.

### Example 3

*Cuminum cyminum* seeds (100 gm) were ground to a coarse powder. Coarse powder was extracted with deionised water at 98 ± 1°C for 2 hrs. Extraction process was repeated four times using total water (200 +3x 100 mL four extractions). All the four extracts were centrifuged, followed by vacuum filtration through celite bed. The clear filtrate was lyophilized to get a greenish yellow amorphous powder (yield 17.0 gm , 17%). Aqueous extract residue was dissolved in deionised water (100 mL) and partitioned between n-BuOH (6 x 100 mL) and H₂O. The n-BuOH extract was concentrated on a rotavapour under reduced pressure at 65°C (residue 2.3 gm). n-BuOH free aqueous extract was freeze dried (residue 13.9 gm). The aqueous residue was dissolved in HPLC grade H₂O (15 mg/mL) and subjected to further purification by preparative HPLC under following conditions:

| | |
|---|---|
| Column | : RP-18, length 10 cmx2 cartridge with guard column |
| Column dia | : 1.5 cm |
| Sample concentration | : 15 mg/mL |
| Injection volume | : 4 mL |
| Mobile phase | : CH₃CN: H₂O (1:9) |
| Flow rate | : 10 mL/min. |
| λmax | : 271 nm |
| Run Time | : 50 minutes |

Pooled target fraction was concentrated under reduced pressure and crystallized from H₂O : EtOH to afford a yellow powder 110 mg , compound Rf 0.28 , solvent system B : A : W (4 : 1 : 5) and was identified as 3',5-dihydroxy flavone 7-O-β-D-galacturonide-4'-O-β-D-glucopyranoside.

### Example 4 :

*Cuminum cyminum* seeds (0.5 kg) were ground to a coarse powder. Coarse powder was defatted with pet. ether 60-80 (1.0 litre) by Soxhlet extraction for 8 hrs. Pet. ether extract was discarded. The marc was dried and then extracted with EtOH (1.0 litre) by Soxhlet extraction for 16 hrs. The EtOH extract was also discarded. The marc was then extracted with 50% aqueous EtOH at room temperature for 16 hrs each time (Total solvent used 1 litre + 4 x 0.5 litre, five extractions). All the five extracts were pooled , concentrated on a rotavapour (residue 67 gm). This residue was dissolved in a minimum quantity of water and adsorbed on silica gel 60-120 mesh (125 gm). A glass column of 1.5 inch dia was packed with silica gel 60-120 mesh (100 gm) in EtOH. The adsorbed extract was charged in the column. Elution was carried out with solvents by gradually increasing the %age of H₂O. Each fraction of 50 mL was collected. The fractions (148-190) eluated in 10% H₂O in EtOH were pooled and subjected to further purification by preparative HPLC using following conditions.

| | |
|---|---|
| Column | :RP-18, length 10 cmx2 cartridge with guard column |
| Column dia | :2.5 cm |
| Sample concentration | :15 mg/mL |
| Injection volume | : 4 mL |
| Mobile phase | :CH₃CN: H₂O (1:9) |
| Flow rate | :10 mL/min. |
| λmax | :271 nm |
| Run Time | :50 minutes |

Pooled target fraction was concentrated under reduced pressure and crystallized from H₂O : EtOH to afford a yellow powder (60 mg) soluble in water, compound Rf 0.28 , solvent system B : A : W (4 : 1 : 5) and was identified as 3',5 -dihydroxyflavone 7-O-β-D-galacturonide-4'-O-β-D-glucopyranoside.

### Example 5.

List of drugs cited as some of the examples for the purpose of the present invention.

| | **Categories** | **Drugs** |
|---|---|---|
| **I** | **Antibiotics** | **Fluoroquinolones:** |
| | | Cipro-, Nor-, P-, and 0-floxacins |
| | | **Macrolides:**Erythro-,Roxythro-,and Azithromycin |
| | | **Cephalosporins:** Cefixime,Cefalexin, Cefadroxil, Cefatrioxone |
| | | **Penicillins:** moxycillin, Cloxacillin |
| | | **Aminoglycosides:** Amikacin, Kanamycin |
| II. | **Antifungal** | Fluconazole, Amphotericin B, Ketoconazole |
| III. | **Anti-viral** | Acyclovir, Zidovudine |
| IV. | **Anti-cancer** | Methotrexate, 5-Fluorouracil, Doxorubicin Cisplatin |
| V. | **Cardiovascular** | Amlodipin ,Lisinopril, Atenolol |
| VI. | **CNS** | Alprazolam, Haloperidol |
| VI. | **Anti-inflammatory/ antiarthritic (NSAID)** | Diclofenac Piroxicam, Nimesulide, Rofecoxib |
| VII. | **Anti-TB/ Antileprosy** | Rifampicin Ethionamide, Isoniazid, Cycloserine, Dapsone,Pyrazinamide, Ethambutol |
| VIII. | **Anti histamines/ respiratory disorders** | Salbutamol, Theophylline, Bromhexine, Loratidine |
| IX. | **Corticosteroids** | Prednisolone, Dexamethasone, Betamethasone |
| X. | **Immunosuppressants** | Cyclosporin A, Tacrolimus Mycophenolate mofetil |
| XI. | **Antiulcer** | Ranitidine, Cimetidine, Omeprazole |

### Example 5 (i): Antibiotics:

**(a) Fluroquinolones**

| Drug | % Enhancement in bioavailability | |
|---|---|---|
| | Active molecule | Fraction* |
| Ciprofloxacin | 65 | 130 |
| P-floxacin | 55 | 137 |
| O-floxacin | 70 | 103 |
| Norfloxacin | 45 | 55 |

**(b) Macrolides**

| Drug | % Enhancement in bioavailability | |
|---|---|---|
| | Active molecule | Fraction* |
| Erythromycin | 70 | 80 |
| Roxythromycin | 65 | 105 |
| Azithromycin | 82 | 115 |

**(c) Cephalosporins**

| Drug | % Enhancement in bioavailability | |
|---|---|---|
| | Active molecule | Fraction* |
| Cefalexin | 70 | 105 |
| Cefadroxil | 85 | 120 |
| Cefatrioxone | 75 | 100 |
| Cefixime | nil | Nil |

**(d) Penicillins**

| Drug | % Enhancement in bioavailability | |
|---|---|---|
| | Active molecule | Fraction* |
| Amoxycillin | 68 | 105 |
| Cloxacillin | 77 | 105 |

**(d) Aminoglycosides:**

| Drug | % Enhancement in bioavailability | |
|---|---|---|
| | Active molecule | Fraction* |
| Amikacin | 76 | 87 |
| Kanamycin | Nil | 35 |

**5 (ii) Antifungal**

| Drug | % Enhancement in bioavailability | |
|---|---|---|
| | Active molecule | Fraction* |
| Fluconazole | 110 | 105 |
| Amphotericin B | 95 | 90 |
| Ketoconazole | 77 | 85 |

**5 (iii) Anti-viral**

| Drug | % Enhancement in bioavailability | |
|---|---|---|
| | Active molecule | Fraction* |
| Acyclovir | 89 | 110 |
| Zidovudine | 120 | 135 |

**5.(iv) CNS drugs:**

| Drug | % Enhancement in bioavailability | |
|---|---|---|
| | Active molecule | Fraction* |
| Alprazolam | 70 | 75 |
| Haloperidol | 72 | 60 |

**5. (v) Anti-cancer**

| Drug | % Enhancement in bioavailability | |
|---|---|---|
| | Active molecule | Fraction* |
| Methotrexate | 95 | 140 |
| 5-Fluorouracil | 110 | 240 |
| Doxorubicin | 78 | 90 |
| Cisplatin | 65 | 95 |

**5. (vi) Cardiovascular:**

| Drug | % Enhancement in bioavailability | |
|---|---|---|
| | Active molecule | Fraction* |
| Amlodipine | 80 | 130 |
| Lisinopril | Nil | Nil |
| Atenolol | 75 | 110 |
| Propranolol | 85 | 140 |

**5. (vii) Anti-inflammatory/ antiarthritic:**

| Drug | % Enhancement in bioavailability | |
|---|---|---|
| | Active molecule | Fraction* |
| Diclofenac | 105 | 125 |
| Piroxicam | 76 | 100 |
| Nimesulide | 90 | 115 |
| Rofecoxib | 43 | 70 |

**5. (viii) Anti-TB/ Antileprosy drugs:**

| Drug | % Enhancement in bioavailability | |
|---|---|---|
| | Active molecule | Fraction* |
| Rifampicin | 90 | 170 |
| Isoniazid | Nil | 30 |
| Pyrazinamide | Nil | Nil |
| Ethambutol | Nil | Nil |
| Dapsone | 67 | 93 |
| Ethionamide | 120 | 110 |
| Cycloserine | 85 | 110 |

**5.(ix) Anti-histamines/respiratory disorders:**

| Drug | % Enhancement in bioavailability | |
|---|---|---|
| | Active molecule | Fraction* |
| Salbutamol | 98 | 75 |
| Theophylline | 75 | 95 |
| Bromhexine | Nil | 35 |
| Loratidine | 62 | 45 |

**5. (x) Corticosteroids:**

| Drug | % Enhancement in bioavailability | |
|---|---|---|
| | Active molecule | Fraction* |
| Prednisolone | 46 | 57 |
| Dexamethasone | 67 | 60 |
| Betamethasone | 65 | 50 |

**5. (xi) Immunosuppressants:**

| Drug | % Enhancement in bioavailability | |
|---|---|---|
| | Active molecule | Fraction* |
| Cyclosporin A | 135 | 170 |
| Tacrolimus | 90 | 110 |
| Mycophenolate Mofeit | Nil | Nil |

**5. (xii) Anti-ulcer**

| Drug | % Enhancement in bioavailability. | |
|---|---|---|
| | Active molecule | Fraction* |
| Ranitidine | 95 | 95 |
| Cimetidine | 76 | 70 |
| Omeprazole | 72 | 87 |

### Example 6: Herbal formulations :

| Drug | %Enhancement in bioavailability/ bioefficacy | |
|---|---|---|
| | Active molecule | Fraction* |
| *Echinacea* | 77 | 147 |
| *Tinospora cordifolia* | 102 | 185 |
| *Picrorrhiza kurroa* | 68 | 180 |
| *Aegles marmelos* | Nil | Nil |
| *Andrographis paniculata* | Nil | 190 |
| *Emblica ribes* | 67 | 90 |
| *Asparagus racemosus* | 78 | 145 |
| *Terminalia chebula* | 45 | 85 |
| *Withania somnifera* | Nil | Nil |
| *Centella asiatica* | 82 | 60 |

### C. Nutraceuticals

| | Category |
|---|---|
| I. | Vitamins |
| | Vitamin A |
| | Vitamin E |
| | Vit.B1 |
| | Vit. B6 |
| | Vit B12 |
| | Vit. C |
| | Folic acid |

| II | Antioxidants |
|---|---|
| | β-Carotene |
| | Silymarin |
| | Selenium |
| | Lycopene |
| | Ellagiogallotannins |

| III | Natural herbal products |
|---|---|
| | Curcumin |
| | Boswellic acids |
| | Rutin |

| IV | Essential nutritional components |
|---|---|
| | Methionine |
| | Lysine |
| | Leucine |
| | Valine |
| | Isoleucine |
| | Zinc |
| | Calcium |
| | Glucose |
| | Potassium |
| | Copper |
| | Iron |

| | |
|---|---|
| *Fraction data shown for reference. | |

Thus, in a method for enhancing bioavailability of drugs / nutraceuticals, said method consisting of admixing to the drug / nutraceutical an effective amount of an active molecule of formula 1 to enhance the pharmaceutical effect of said active drug / nutraceutical without any harmful side effect:
the bioavailability of the antibiotic is enhanced by 45 to 85% when the same is mixed with the active molecule of formula 1;
the bioavailability of the antifungal drug is enhanced by 77 to 110% when the same is mixed with the active molecule of formula 1;
the bioavailability of the antiviral drug is enhanced by 89 to 120% when the same is mixed with the active molecule of formula 1;
the bioavailability of the CNS drug is enhanced by 70 to 72% when the same is mixed with the active molecule of formula 1;
the bioavailability of the anti-cancer drug is enhanced by 65 to 110% when the same is mixed with the active molecule of formula 1;
the bioavailability of the cardiovascular disorder drug is enhanced by 75 to 85% when the same is mixed with the active molecule of formula 1;
the bioavailability of the anti-inflammatory / antiarthritic drug is enhanced by of 43 to 105% when the same is mixed with the active molecule of formula 1;
the bioavailability of the anti-TB / antileprosy drug is enhanced by of 67 to 120% when the same is mixed with the active molecule of formula 1;
the anti-histamines / respiratory disorder drug is enhanced by of 62 to 98% when the same is mixed with the active molecule of formula 1;
the bioavailability of corticosteroids is enhanced by of 46 to 67% when the same is mixed with the active molecule of formula 1;
the bioavailability of immunosuppressants is enhanced by of 90 to 135% when the same is mixed with the active molecule of formula 1;
the bioavailability of anti-ulcer drugs is enhanced by 72 to 85% when the same is mixed with the active molecule of formula 1; and
the bioavailability of herbal formulation is enhanced by 45 to 102% when the same is mixed with the active molecule of formula 1.

## Claims

1. A composition having enhanced bioavailability of drugs / nutraceuticals, said composition consisting of an active drug / nutraceutical and an active molecule of formula 1 as bioenhancer in an amount effective to enhance the pharmaceutical effect of said active drug / nutraceutical without any harmful side effect.

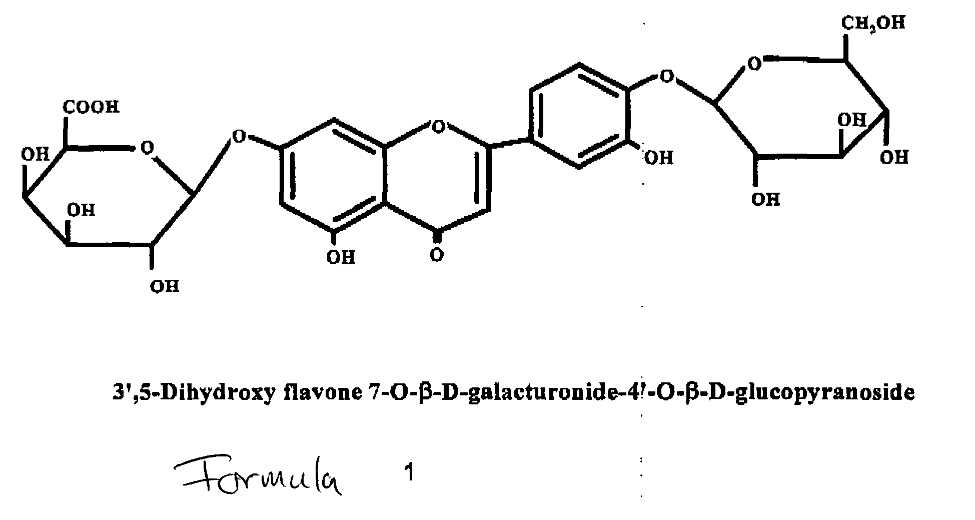

2. A composition according to claim 1, wherein w/w ratio of the bioenhancer to the drug / nutraceutical is in the range of 0.1 to 300.

3. A composition according to claim 1, wherein said active drug is selected from the group consisting of antibiotics, antifungal drugs, antiviral drugs, anticancer drugs, cardiovascular disorder drugs, CNS disorder drugs, antiinflammatory / antiarthritic drugs, anti-TB / anti-leprosy drugs, anti-histamines / respiratory disorder drugs, corticosteroids, immuno-suppressants and anti-ulcer drugs.

4. A composition according to claim 3, wherein said antibiotic is selected from the group consisting of fluroquinolones, macrolides, cephalosporins, penicillins and aminoglycosides.

5. A composition according to claim 4, wherein said fluroquinolone is selected from the group consisting of ciprofloxacin, p-floxacin, o-floxacin and norfloxacin, said macrolide is selected from the group consisting of erythromycin, roxythromycin and azithromycin, said cephalosporin is selected from the group consisting of cefalexin, cefadroxil and cefatrioxone, said penicillin is selected from the group consisting of amoxycillin and cloxacillin, and said aminoglycoside is amikacin.

6. A composition according to claim 3, wherein said antifungal drug is selected from the group consisting of fluconazole, amphotericin B and ketoconazole.

7. A composition according to claim 3, wherein said antiviral drug is selected from the group consisting of acyclovir and zidovudine.

8. A composition according to claim 3, wherein said CNS disorder drug is selected from the group consisting of alprazolam and haloperidol.

9. A composition according to claim 3, wherein said anti-cancer drug is selected from the group consisting of methotrexate, 5-fluorouracil, doxorubicin and cisplatin.

10. A composition according to claim 3, wherein said cardiovascular disorder drug is selected from the group consisting of amlodipine, atenolol and propranolol.

11. A composition according to claim 3, wherein said anti-inflammatory / antiarthritic drug is selected from the group consisting of diclofenac, piroxicam, nimesulide and rofecoxib.

12. A composition according to claim 3, wherein said anti-TB / antileprosy drug is selected from the group consisting of rifampicin, dapsone, ethionamide and cycloserine.

13. A composition according to claim 3, wherein said anti-histamines / respiratory disorder drug is selected from the group consisting of salbutamol, theophylline and loratidine.

14. A composition according to claim 3, wherein said corticosteroid is selected from the group consisting of prednisolone, dexamethasone and betamethasone.

15. A composition according to claim 3, wherein said immunosuppressant is selected from the group consisting of cyclosporin A and tacrolimus.

16. A composition according to claim 3, wherein said anti-ulcer drug is selected from the group consisting of ranitidine, cimetidine and omeprazole.

17. A composition according to claim 1, wherein the nutraceutical is selected from the group consisting of vitamins, antioxidants, natural herbal products, herbal formulations and essential nutritional components.

18. A composition according to claim 17, wherein said vitamin is selected from the group consisting of Vitamin A, E, B1, B6, B12, C and folic acid.

19. A composition according to claim 17, wherein said antioxidant is selected from the group consisting of β-carotene, silymarin, selenium, lycopene and ellagiogallotannins.

20. A composition according to claim 17, wherein said natural herbal product is selected from the group consisting of curcumin, boswellic acids and rutin.

21. A composition according to claim 17, wherein herbal formulation is selected from the group consisting of echinacea, tinospora cordifolia, pierorrhiza kurroa, emblica ribes, asparagus racemosus, terminalia chebula and centella asiatica.

22. A composition according to claim 17, wherein said nutritional component is selected from the group consisting of methionine, lysine, leucine, valine, isoleucine, zinc, calcium, glucose, potassium, copper and iron.

23. A composition according to claim 1, wherein said composition is to be administered orally or intramuscularly and is also relevant to animal health.

24. A method for enhancing bioavailability of drugs / nutraceuticals, said method consisting of admixing to the drug / nutraceutical an active molecule of formula 1 as bioenhancer in an amount effective to enhance the pharmaceutical effect of said active drug / nutraceutical without any harmful side effect.

## Patentansprüche

1. Eine Zusammensetzung mit erhöhter Bioverfügbarkeit von Arzneistoffen/Nutrazeutika, wobei die Zusammensetzung besteht aus einem aktiven Arzneistoff/Nutrazeutikum und einem aktiven Molekül der Formel 1 als Bioverstärker in einer effektiven Menge um den pharmazeutischen Effekt des aktiven Arzneistoffs/Nutrazeutikums ohne schädliche Nebenwirkung zu verstärken.

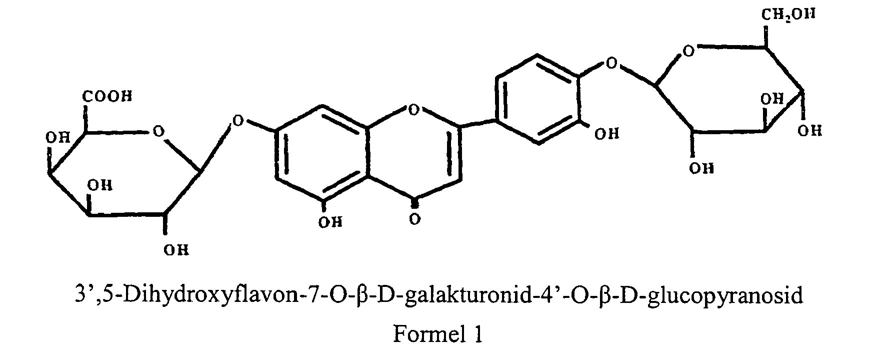

2. Eine Zusammensetzung nach Anspruch 1, wobei das Gew./Gew.-Verhältnis des Bioverstärkers zu dem Arzneistoff/Nutrazeutikum im Bereich von 0,1 bis 300 liegt.

3. Eine Zusammensetzung nach Anspruch 1, wobei der aktive Arzneistoff ausgewählt wird aus der Gruppe bestehend aus Antibiotika, Arzneistoffen gegen Pilzbefall, antiviralen Arzneistoffen, Antikrebsmitteln, Arzneistoffen gegen Herz-Kreislauf-Erkrankung, Arzneistoffen gegen Erkrankung des ZNS (Zentral-Nervensystems), entzündungshemmenden/antiarthritischen Arzneistoffen, Anti-Tuberkulose-/Anti-Lepra-Arzneistoffen, Antihistaminen/Arzneimitteln gegen Atemwegserkrankungen, Corticosteroiden, Immunosuppressiva und Arzneistoffen gegen Ulzera.

4. Eine Zusammensetzung nach Anspruch 3, wobei das Antibiotikum ausgewählt wird aus der Gruppe bestehend aus Fluorchinolonen, Makroliden, Cephalosporinen, Penicillinen und Aminoglycosiden.

5. Eine Zusammensetzung nach Anspruch 4, wobei d-Fluorchinolon ausgewählt wird aus der Gruppe bestehend aus Ciprofloxacin, p-Floxacin, o-Floxacin und Norfloxacin, das Makrolid ausgewählt wird aus der Gruppe bestehend aus Erythromycin, Roxithromycin und Azithromycin, das Cephalosporin ausgewählt wird aus der Gruppe bestehend aus Cefalexin, Cefadroxil und Ceftriaxon, das Penicillin ausgewählt wird aus der Gruppe bestehend aus Amoxicillin und Cloxacillin, und das Aminoglycosid Amikacin ist.

6. Eine Zusammensetzung nach Anspruch 3, wobei der Arzneistoff gegen Pilzbefall ausgewählt wird aus der Gruppe bestehend aus Fluconazol, Amphotericin B und Ketoconazol.

7. Eine Zusammensetzung nach Anspruch 3, wobei der antivirale Arzneistoff ausgewählt wird aus der Gruppe bestehend aus Aciclovir und Zidovudin.

8. Eine Zusammensetzung nach Anspruch 3, wobei der Arzneistoff gegen Erkrankungen des ZNS ausgewählt wird aus der Gruppe bestehend aus Alprazolam und Haloperidol.

9. Eine Zusammensetzung nach Anspruch 3, wobei das Antikrebsmittel ausgewählt wird aus der Gruppe bestehend aus Methotrexat, 5-Fluorouracil, Doxorubicin und Cisplatin.

10. Eine Zusammensetzung nach Anspruch 3, wobei der Arzneistoff gegen Herz-Kreislauf-Erkrankung ausgewählt wird aus der Gruppe bestehend aus Amlodipin, Atenolol und Propranolol.

11. Eine Zusammensetzung nach Anspruch 3, wobei der entzündungshemmende/antiarthritische Arzneistoff ausgewählt wird aus der Gruppe bestehend aus Diclofenac, Piroxicam, Nimesulid und Rofecoxib.

12. Eine Zusammensetzung nach Anspruch 3, wobei der Anti-Tuberkulose-/Anti-Lepra-Arzneistoff ausgewählt wird aus der Gruppe bestehend aus Rifampicin, Dapson, Ethionamid und Cycloserin.

13. Eine Zusammensetzung nach Anspruch 3, wobei die Antihistaminika/der Arzneistoff gegen Atemwegserkrankungen ausgewählt wird aus der Gruppe bestehend aus Salbutamol, Theophyllin und Loratadin.

14. Eine Zusammensetzung nach Anspruch 3, wobei das Corticosteroid ausgewählt wird aus der Gruppe bestehend aus Prednisolon, Dexamethason und Betamethason.

15. Eine Zusammensetzung nach Anspruch 3, wobei das Immunsuppressivum ausgewählt wird aus der Gruppe bestehend aus bestehend aus Ciclosporin A und Tacrolimus.

16. Eine Zusammensetzung nach Anspruch 3, wobei der Arzneistoff gegen Ulzera ausgewählt wird aus der Gruppe bestehend aus Ranitidin, Cimetidin und Omeprazol.

17. Eine Zusammensetzung nach Anspruch 1, wobei das Nutrazeutikum ausgewählt wird aus der Gruppe bestehend aus Vitaminen, Antioxidantien, natürlichen pflanzlichen Produkten, pflanzlichen Formulierungen und essentiellen Nahrungskomponenten.

18. Eine Zusammensetzung nach Anspruch 17, wobei das Vitamin ausgewählt wird aus der Gruppe bestehend aus Vitamin A, E, B1, B6, B12, C und Folsäure.

19. Eine Zusammensetzung nach Anspruch 17, wobei das Antioxidans ausgewählt wird aus der Gruppe bestehend aus β-Carotin, Silymarin, Selen, Lycopin und Ellagiogallotanninen.

20. Eine Zusammensetzung nach Anspruch 17, wobei das Produkt aus natürlichen Pflanzen ausgewählt wird aus der Gruppe bestehend aus Curcumin, Boswelliasäuren und Rutin.

21. Eine Zusammensetzung nach Anspruch 17, wobei die pflanzliche Formulierung ausgewählt wird aus der Gruppe bestehend aus Echinacea, Tinospora cordifolia, Picrorrhiza kurroa, Emblica ribes, Asparagus racemosus, Terminalia chebula und Centella asiatica.

22. Eine Zusammensetzung nach Anspruch 17, wobei die Nahrungskomponente ausgewählt wird aus der Gruppe bestehend aus Methionin, Lysin, Leucin, Valin, Isoleucin, Zink, Calcium, Glukose, Kalium, Kupfer und Eisen.

23. Eine Zusammensetzung nach Anspruch 1, wobei die Zusammensetzung oral oder intramuskulär zu verabreichen ist und relevant für Tiergesundheit ist.

24. Ein Verfahren zur Steigerung der Bioverfügbarkeit von Arzneistoffen/Nutrazeutika, wobei das Verfahren besteht im Beimischen eines aktiven Moleküls der Formel 1 als Bioverstärker zu dem Arzneistoff/Nutrazeutikum in einer effektiven Menge, um die pharmazeutische Wirkung des aktiven Arzneistoffes/Nutrazeutikums ohne schädliche Nebenwirkungen zu verstärken.

## Revendications

1. Composition ayant une biodisponibilité accrue de médicaments/nutraceutiques, ladite composition consistant en un médicament/nutraceutique actif et en une molécule active de la formule 1 en qualité de bioactivateur en une quantité suffisante pour stimuler l'effet pharmaceutique dudit médicament/nutraceutique actif sans effet secondaire nuisible.

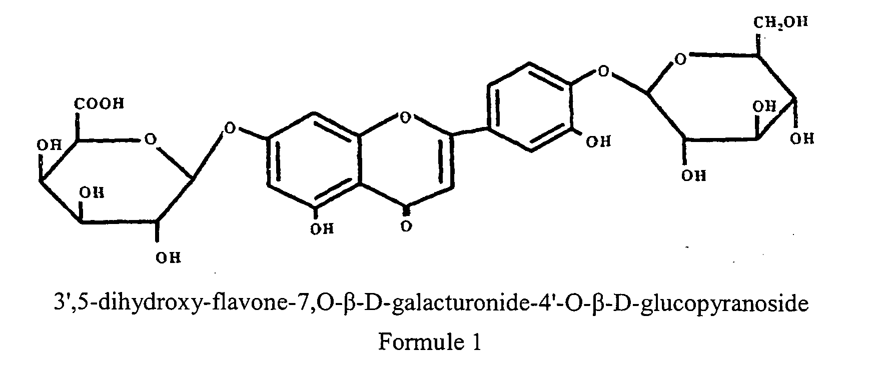

2. Composition selon la revendication 1, dans laquelle le rapport p/p du bioactivateur au médicament/nutraceutique, est dans la plage de 0,1 à 300.

3. Composition selon la revendication 1, dans laquelle ledit médicament actif est sélectionné parmi le groupe consistant en antibiotiques, médicaments antifongiques, médicaments antiviraux, médicaments anticancéreux, médicaments contre les troubles cardiovasculaires, médicaments contre les troubles du SNC, médicaments anti-inflammatoires/anti-arthritiques, médicaments antituberculose/contre la lèpre, médicaments antihistaminiques/contre les troubles respiratoires, corticostéroïdes, immunosuppresseurs et médicaments anti-ulcéreux.

4. Composition selon la revendication 3, dans laquelle ledit antibiotique est sélectionné parmi le groupe consistant en fluoroquinolones, macrolides, céphalosporines, pénicillines et aminoglycosides.

5. Composition selon la revendication 4, dans laquelle ladite fluoroquinolone est sélectionnée parmi le groupe consistant en ciprofloxaxine, p-floxacine, o-floxacine et norfloxacine, ledit macrolide est sélectionné parmi le groupe consistant en érythromycine, roxythromycine et azithromycine, ladite céphalosporine est sélectionnée parmi le groupe consistant en céfalexine, céfadroxil et céfatrioxone, ladite pénicilline est sélectionnée parmi le groupe consistant en amoxycilline et cloxacilline et ledit aminoglycoside est l'amikacine.

6. Composition selon la revendication 3, dans laquelle ledit médicament antifongique est sélectionné parmi le groupe consistant en fluconazole, amphotéricine B et kétoconazole.

7. Composition selon la revendication 3, dans laquelle ledit médicament antiviral est sélectionné parmi le groupe consistant en acyclovir et en zidovudine.

8. Composition selon la revendication 3, dans laquelle ledit médicament contre les troubles du SNC est sélectionné parmi le groupe consistant en alprazolam et en halopéridol.

9. Composition selon la revendication 3, dans laquelle ledit médicament anticancéreux est sélectionné parmi le groupe consistant en méthotrexate, 5-fluorouracile, doxorubicine et cisplatine.

10. Composition selon la revendication 3, dans laquelle ledit médicament contre les troubles cardiovasculaires est sélectionné parmi le groupe consistant en amlodipine, atenolol et propanolol.

11. Composition selon la revendication 3, dans laquelle ledit médicament anti-inflammatoire/anti-arthritique est sélectionné parmi le groupe consistant en diclofénac, piroxicam, nimésulide et rofécoxib.

12. Composition selon la revendication 3, dans laquelle ledit médicament antituberculose/contre la lèpre est sélectionné parmi le groupe consistant en rifampicine, dapsone, éthionamide et cyclosérine.

13. Composition selon la revendication 3, dans laquelle ledit médicament antihistamique/contre les troubles respiratoires est sélectionné parmi le groupe consistant en salbutamol, théophylline et loratidine.

14. Composition selon la revendication 3, dans laquelle ledit corticostéroïde est sélectionné parmi le groupe consistant en prednisolone, dexaméthazone et bétaméthasone.

15. Composition selon la revendication 3, dans laquelle ledit immunosuppresseur est sélectionné parmi le groupe consistant en cyclosporine A et en tacrolimus.

16. Composition selon la revendication 3, dans laquelle ledit médicament anti-ulcéreux est sélectionné parmi le groupe consistant en ranitidine, cimétidine et oméprazole.

17. Composition selon la revendication 1, dans lequel le nutraceutique est sélectionné parmi le groupe consistant en vitamines, antioxydants, produits naturels à base de plantes, formulations à base de plantes et composants nutritifs essentiels.

18. Composition selon la revendication 17, dans laquelle ladite vitamine est sélectionnée parmi le groupe consistant en vitamine A, E, B1, B6, B12, C et en acide folique.

19. Composition selon la revendication 17, dans laquelle ledit antioxydant est sélectionné parmi le groupe consistant en β-carotène, silymarine, sélénium, lycopène et ellagiogallotanins.

20. Composition selon la revendication 17, dans laquelle ledit produit naturel à base de plantes est sélectionné parmi le groupe consistant en curcumin, acides boswelliques et rutine.

21. Composition selon la revendication 17, dans laquelle la formulation à base de plantes est sélectionnée parmi le groupe consistant en *Echinacea, Tinospora cordifolia, Picrorrhiza kurroa, Emblica ribes, Asparagus racemosus, Terminalia chebula* et *Centella asiatica.*

22. Composition selon la revendication 17, dans laquelle ledit composant nutritif est sélectionné parmi le groupe consistant en méthionine, lysine, leucine, valine, isoleucine, zinc, calcium, glucose, potassium, cuivre et fer.

23. Composition selon la revendication 1, où ladite composition doit être administrée par voie orale ou intramusculaire et est aussi pertinente à la santé animale.

24. Méthode pour accroître la biodisponibilité de médicaments/nutraceutiques, ladite méthode consistant à ajouter à et mélanger avec le médicament/nutraceutique, une molécule active de la formule 1 en qualité de bioactivateur en une quantité efficace pour stimuler l'effet pharmaceutique dudit médicament/nutraceutique actif sans aucun effet secondaire nuisible.
